# EUROPEAN PATENT APPLICATION

(11) **EP 1 249 502 A2**
(43) Date of publication of application: **16.10.2002**
(21) Application number: 02007925.7
(22) Date of filing: 09.04.2002
(51) Int. Cl.: C12Q 1/68, C12N 15/10, G01N 15/14, G06F 19/00

(54) **Beads, preparing method for the same, flow cytometer and program**

(30) Priority: 09.04.2001 JP 2001109940
(71) Applicant: Hitachi Software Engineering Co., Ltd., Yokohama-shi, Kanagawa 231-8475 (JP)
(72) Inventor: Nakao, Motonao, Hitachi Software Engineering Co., Yokohama-shi, Kanagawa 231-8475 (JP); Tatsuguchi, Koichi, Hitachi Softw. Engineering Co., Yokohama-shi, Kanagawa 231-8475 (JP)
(74) Representative: Liesegang, Roland, Dr.-Ing.

(57) **Abstract**

Disclosed are beads, which are chemically stable even at a normal temperature, are stable without being disintegrated even if being exposed to light, can emit a plurality of fluorescence excited by irradiating a light of single wavelength, and can be identified by a flow cytometer. A method for preparing the beads, a flow cytometer and a program for preparing the beads are also disclosed. By dyeing semiconductor nanoparticles as a fluorescent reagent to polystyrene beads, beads are identified. At the same time, semiconductor nanoparticles of a different fluorescence wavelength are also used for detection of reporter, enabling SNP specification of gene, monitoring or finding of the concentration of biopolymer such as a protein. The semiconductor nanoparticles have a feature that the fluorescence wavelength may vary by controlling the particle size and that they have a high durability compared to an ordinary fluorescent reagent since they are a semiconductor.

## Description

### BACKGROUND OF THE INVENTION

### 1. Field of the Invention

The present invention relates to beads suitably used as a label of a probe or the like for detecting single nucleotide polymorphism (SNP), a method of preparing the beads, a flow cytometer for detecting the beads, and a program to be used for the flow cytometer.

### 2. Description of the Related Art

Conventionally, flow cytometry is known as a tool to examine shapes of particle substances existent in cells, for example, red blood cells or white blood cells.

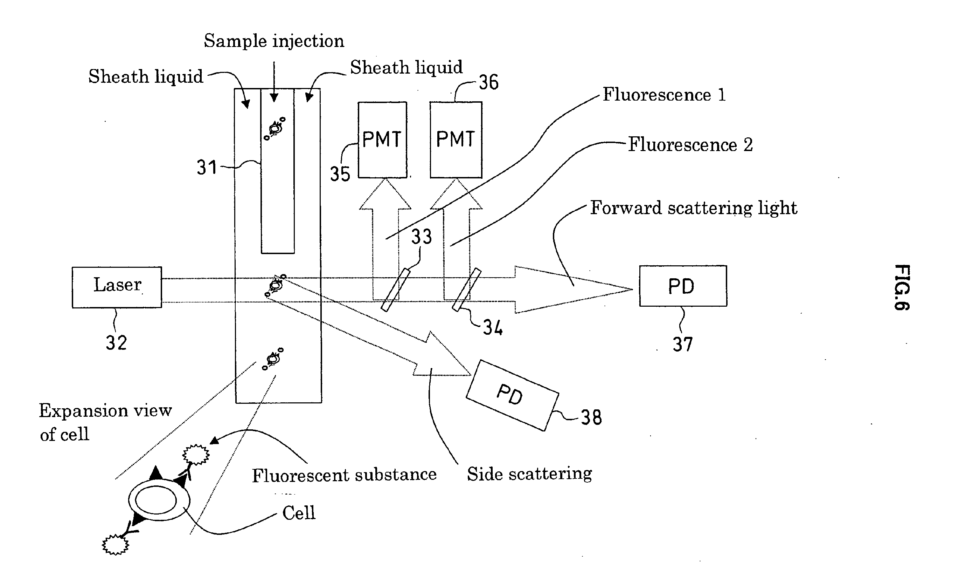

FIG. 6 is a diagram showing a schematic configuration of conventional flow cytometry. A solution containing particles sample is injected into a cylindrical tubule 31 (sample injection). Then, when the sample flows out of the tubule, the flow of the sample is throttled due to a hydrodynamic throttling caused by a sheath liquid flowing around the sample. Then, the flow rate of the sample is adjusted such that each particle of these particles substances do not overlap each other at the time of measurement. Subsequently, the particle sample in the narrow tubule is irradiated with a laser beam from a laser 32.

Fluorescence is radiated from a fluorescent substance excited by a laser beam, a fluorescence wavelength is separated by filters 33 and 34, and fluorescence intensity for each fluorescence wavelength is obtained by PMTs (photomultipliers) 35, 36 and the like. In addition, a forward scattering light and a side scattering light are detected by photodiodes 37 and 38.

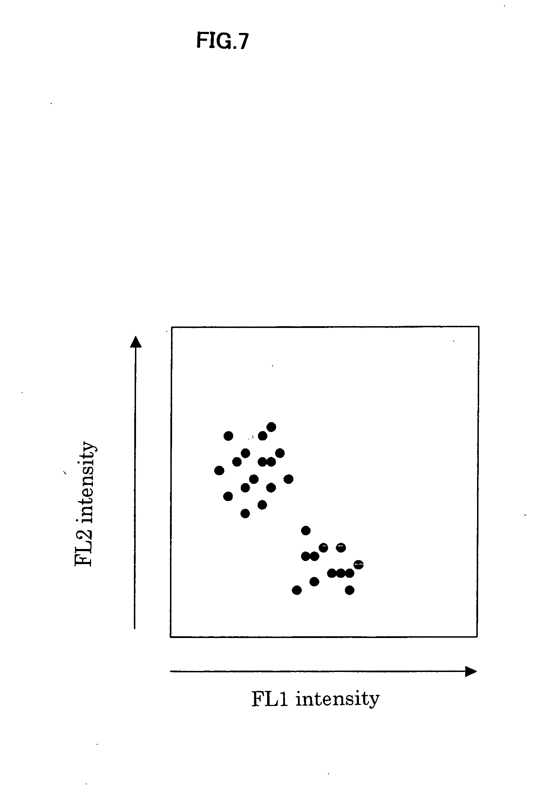

FIG. 7 is a diagram showing a result of measurement using a conventional flow cytometer. The measurement is carried out using fluorescein isothiocyanate (FITC) as a fluorescent substance in FL1 and phycoerythrin (PE) as a fluorescent substance in FL2.

Generally, each fluorescent substance is previously bonded to a protein such as an antibody. Then, by quantitatively measuring fluorescence radiated from an antibody-fluorescent substance reacting on an antigen region by use of an antigen-antibody reaction with the antigen region existent on a surface of a cell, it is possible to indirectly measure a proportion of the antigen existent on the surface of the cell.

Experiments have been recently conducted using the above technologies, in which beads made of polystyrene are used as carriers in place of cells and identification of the beads is performed by use of a technology of flow cytometry. Further, products practically commercialized have been marketed.

The above technology is one in which these beads are dyed with a fluorescent substance having its concentration changed in advance, measurement is carried out in FL1 or FL2 of a flow cytometer, and then the beads are identified by a difference in concentration between FL1 and FL2.

PE and FITC have been so far known as fluorescent substances which have been conventionally used for flow cytometry, and a flow cytometer mounted with a UV laser has recently appeared as a commercial item. Therefore, it has been made possible to excite and measure DAPI (4'-6-diimide-2-phenylindole) or Hoechst Dye (bisbenzimide H33258).

These organic series fluorescence reagents are, however, vulnerable not only to a laser beam, but also to an ordinary sunlight, and inherently have their drawbacks that the substances are disintegrated through exposure to light and fluorescence intensity is reduced.

Therefore, the aforementioned case where beads are dyed with the fluorescence reagents has similar drawbacks. Thus, light shielding and freezing are required to store the reagents, and attention must be paid in this regard.

### SUMMARY OF THE INVENTION

It is an object of the present invention to provide, in light of the problems mentioned above, beads which are chemically stable even at normal temperature, are also stable, without being disintegrated even if being exposed to light, are capable of emitting a plurality of fluorescence by being excited by irradiation of a single-wavelength light, and can be identified by a flow cytometer, as well as to provide a method for preparing beads, a flow cytometer and a program therefor for preparing the beads.

Beads according to the present invention have semiconductor nanoparticles with a particle size of 1 to 10 nm on surfaces thereof.

In addition, the semiconductor nanoparticles are composed of particles having a plurality of particle sizes so as to emit a light having peaks in a plurality of wavelengths by being excited by an electromagnetic wave. Thus, many beads can be identified.

A method of preparing beads according to an aspect of the present invention comprises the steps of: covalent-bonding DNA with surfaces of the beads; and introducing a label as a reporter onto surfaces of the beads by performing reverse transcription reaction using a desired mRNA as a template, the DNA as a primer, and a base as a material, in which at least one kind of base is labeled. Accordingly, it is possible to quantify mRNA which is a target.

A method of preparing beads according to another aspect of the present invention comprises the steps of: covalent-bonding DNA with surfaces of the beads; and introducing a label as a reporter onto surfaces of the beads by performing PCR using a desired mRNA or cDNA as a template, the DNA as a primer, and a base as a material, in which at least one kind of base is labeled. Accordingly, it is possible to quantify mRNA which is expressed only by a small amount.

In the step of the covalent-bonding, a base with a substituent existing at the end thereof is fixed on surfaces of the beads, the beads are filled in a biological reactor, and then the base is further coupled to the substituent, whereby DNA synthesis is performed on solid phase beads. Accordingly, it is generally possible to perform the DNA synthesis and the covalent-bonding of DNA to the beads in a single reactor, thereby increasing production capability of beads chips.

In addition, a flow cytometer according to the present invention is provided with a UV laser emitting a UV laser beam to excite a sample, a plurality of fluorescence detecting means for detecting a plurality of fluorescence emitted from the sample, and switching means for switching output signals from the fluorescence detecting means to either bead identifying signal or a reporter detecting signal.

Further, a program according to the present invention is for allowing a computer to function as a flow cytometer which comprises: a UV laser emitting a UV laser beam to excite a sample; a plurality of fluorescence detecting means for detecting a plurality of fluorescence emitted from the sample, respectively; and switching means for switching output signals from the fluorescence detecting means to either bead identifying signal or a reporter detecting signal.

As described above, the present invention can provide beads which are chemically stable even at normal temperature, are stable without being disintegrated even if being exposed to light, are capable of emitting a plurality of fluorescence by being excited by irradiation of a single-wavelength light, as well as can provide a method of preparing beads, a flow cytometer therefor and a program therefor.

### BRIEF DESCRIPTION OF THE DRAWINGS

FIG. 1 is a diagram showing a relation between mixing ratio of semiconductor nanoparticles and intensity of emitted fluorescence;
FIG. 2 is a schematic drawing showing a method for synthesizing DNA by parallel array method;
FIG. 3 is a diagram showing an example where a different processing is performed depending upon beads;
FIG. 4 is a diagram showing the results of measurement by a UV laser-installed FACS (cell sourcer);
FIG. 5 is a schematic drawing showing a detector capable of changing kind of fluorescence for identification of beads and for detection of reporter;
FIG. 6 is a diagram showing a schematic configuration of a conventional flow cytometry;
FIG. 7 is a diagram showing the result of a measurement using a conventional flow cytometer; and
FIG. 8 is a diagram showing a relationship between fluorescence wavelength of semiconductor nanoparticles (CdSe-ZnS) and the particle size of the nanoparticles.

### DETAILED DESCRIPTION OF THE PREFERRED EMBODIMENTS

Recently, it has been proven that a semiconductor nanoparticles can be produced and that bonding of the semiconductor nanoparticles to a biopolymer is also applicable (Sience, vol. 281, p2013 (1998)). The semiconductor nanoparticles are inorganic compounds having a characteristic that, in its nature, a wavelength of fluorescence varies depending on their particles size.

In addition, since excitation is possible if an energy exceeding a band gap is given for excitation, ordinary semiconductor nanoparticles of various kinds of particle sizes can be excited by a laser outputting a high energy on a UV side.

Various methods have been reported for the method of preparing semiconductor nanoparticles, such as a vapor deposition method, an organic chemical synthesis method, and a preparation method using microwave (refer to the Publication of Japanese Patent No.3005683).

In order to utilize the semiconductor nanoparticles as a fluorescent substance, the semiconductor nanoparticles need to emit a light with a sharp fluorescence wavelength. For that purpose, it is, therefore, necessary to prepare semiconductor nanoparticles of a uniform particle size.

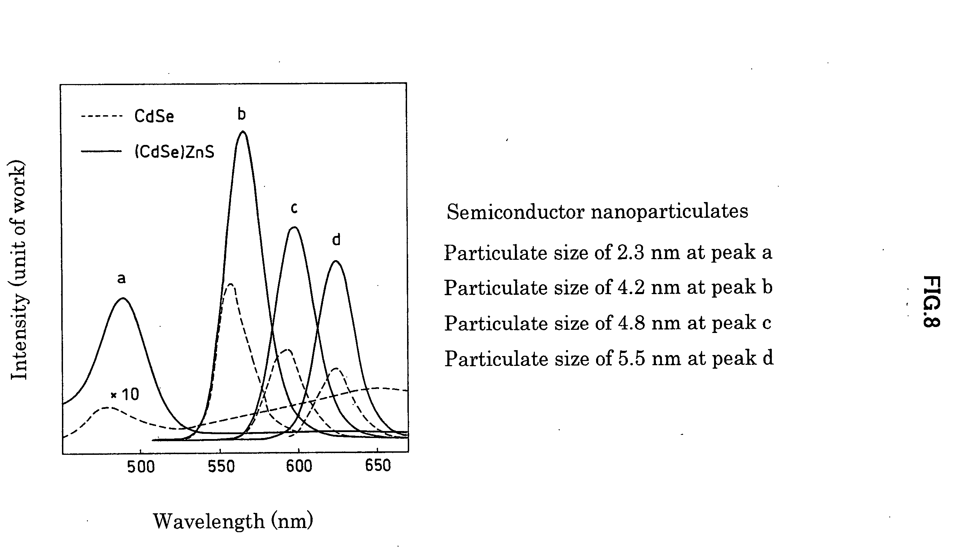

FIG. 8 is a diagram showing a relationship between a fluorescence wavelength of semiconductor nanoparticles (CdSe-ZnS) and a particle size of the nanoparticles.

The present invention is to provide beads having semiconductor nanoparticles prepared on surfaces thereof made of polystyrene by the foregoing methods or the like, which allows a flow cytometer to identify the beads, as well as to provide a method of preparing the beads, the flow cytometer, and a program for allowing a computer to function as the flow cytometer.

Hereinafter, detailed descriptions will be made for an embodiment of the present invention.

First, two kinds of semiconductor nanoparticles with different fluorescence wavelengths (particle sizes of 4.2nm and 5.5nm) are prepared and are bonded to polystyrene beads (a particle size within the range of 0.1µm to 100µm, more preferably within the range of 1µm to 10µm).

In this case, there is no restriction on a material of the beads and a method of bonding the beads.

In this embodiment, the polystyrene beads are dyed with two kinds of semiconductor nanoparticles as fluorescence reagents obtained by dissolving in solvents the semiconductor nanoparticles having mixing ratios thereof varied.

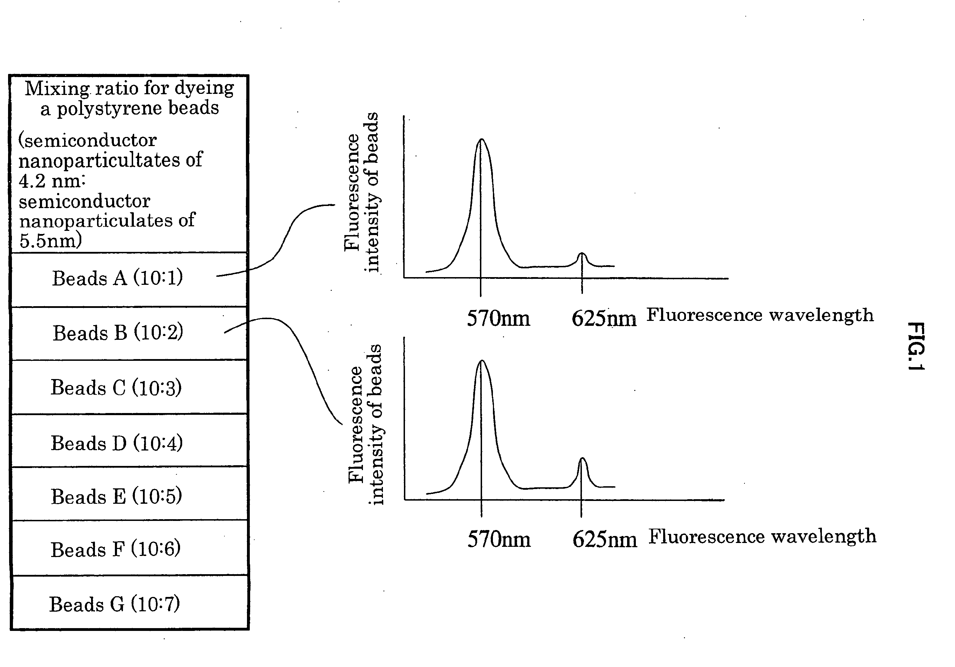

FIG. 1 is a diagram showing a relation between the mixing ratio of the semiconductor nanoparticles and the intensity of emitted fluorescence. The semiconductor nanoparticles with particle size of 4.2nm and the semiconductor nanoparticles with particle size of 5.5nm are mixed. With the mixing ratio varied from 10:1 to 10:7, polystyrene beads A to G are dyed, respectively. The result of measuring beads A (semiconductor nanoparticles of 4.2nm: semiconductor nanoparticles of 5.5nm = 10: 1) and beads B (10: 2) by a flow cytometer is shown. With regard to a peak of the light intensity at a wavelength of 570nm corresponding to the semiconductor nanoparticles of 4.2nm, A and B are almost the same. On the contrary, with regard to a peak of the light intensity at a wavelength of 625nm corresponding to the semiconductor nanoparticles of 5.5nm, the beads B shows a relative intensity relative to the peak of the light intensity at a wavelength of 570nm, which is approximately twice as high as that of the beads A. Thus, using the relative intensity of the peak of the light intensity at 625nm with respect to the peak of the light intensity at 570nm as a signal, the beads may be identified.

Either of four bases A, T, C, G is immobilized to the prepared beads A to G. After the base is immobilized, the beads are filled in the reactor, and DNA synthesis is performed.

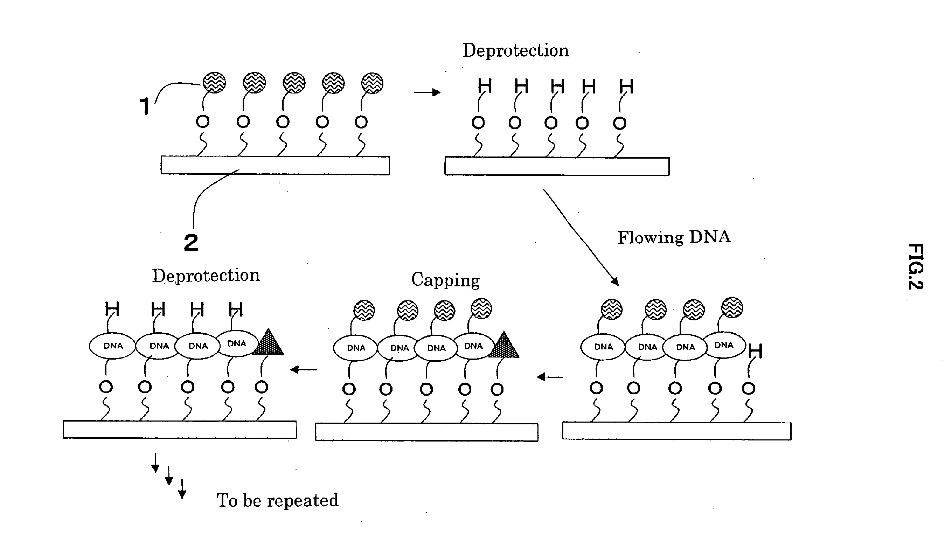

FIG.2 is a schematic drawing showing a method for synthesizing DNA by parallel array method.

In the parallel array method, DNA is synthesized by a series of β-cyanoethyl chemical reaction. The following steps are repeated for each cycle: "deprotection" in which 5'-hydrogen group is freed by removing trityl group 1 from first nucleotide immobilized to an immobilized carrier 2; "coupling" in which the next nucleotide is bonded by flowing DNA; "capping" in which a region that nucleotide failed to be bonded is capped; "oxidization" (not shown) in which a region to which second nucleotide is bonded; and "deprotection" of second nucleotide.

Here, an immobilized carrier 2 is a bead.

As mentioned above, synthesis may be performed on the bead. Alternatively, DNA may be bonded to the bead after DNA is synthesized with an end modified in advance.

The bead with DNA covalent-bonded on a surface thereof through such processes may be subjected to hybridization for a complementary strand with the DNA.

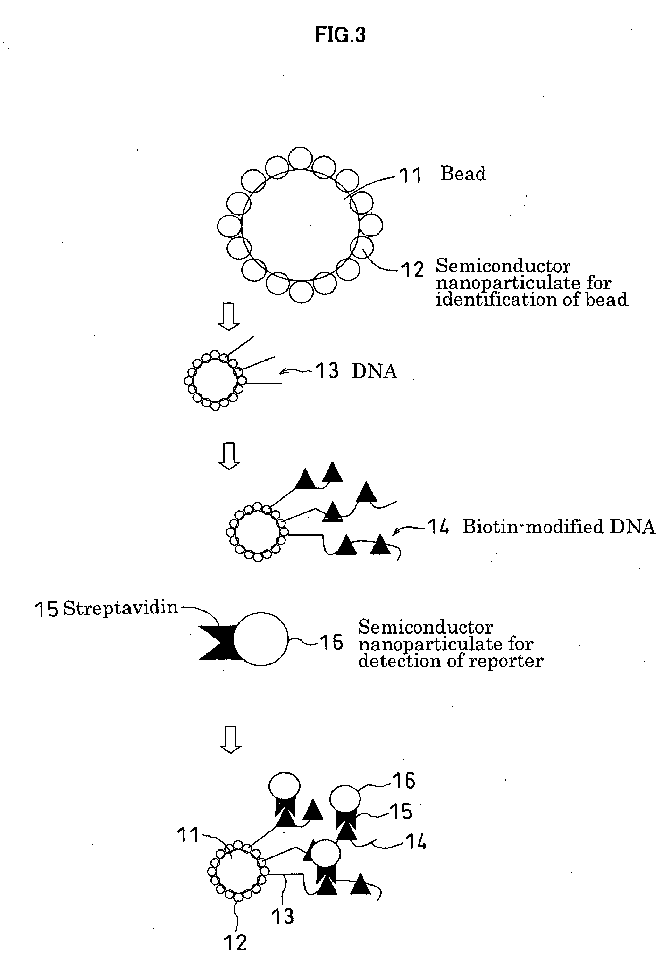

FIG. 3 is a diagram showing an example where a different processing is performed depending upon a bead. For example, an array (5'-CAG GCC AAG TAA CTT CTT CG-3') is synthesized on beads A, and an array (5'-TCC TTC TGC ATC CTG TCG GCA-3') is synthesized on beads B.

The array on beads A is a gene derived from luciferase, and no complementary strand is existent in mRNA expressed in a ordinary human body. In addition, the array on beads B is of a complementary strand with mRNA of β actin gene in a human body, and it is found that hybridization may be performed with mRNA in total RNA of an ordinary human body. β actin gene is widely known as a house keeping gene generally expressed in any cells.

Thus, two kinds of beads dyed by semiconductor nanoparticles 12 for identification of beads, with different DNA 13 immobilized severally thereto are prepared. Then, using a human body derived total RNA and a reverse transcriptase, reverse transcription reaction is performed. dUTP modified with biotin (Biotin-11-dU TP, Amersham Pharmacia RPZ2001) is used for one of the four kinds of dNTP (deoxynucleotide) to be used in this case, and reverse transcription reaction is performed. If a complementary strand of DNA bonded to bead in total RNA is existent, reverse transcription reaction is performed, dUTP modified with biotin (shown in a triangle) is incorporated, and a DNA 14 modified with biotin is bonded.

With the processing up to this stage, beads B are modified with biotin, and beads A are not modified with biotin.

In this stage, if a conjugate in which semiconductor nanoparticles 16 of 2.3nm are bonded to streptavidin 15 is added to a solution in which beads A and beads B exist, beads B are indirectly modified with semiconductor nanoparticles 16 of 2.3nm by bonding of biotin- streptavidin, and beads A are not modified

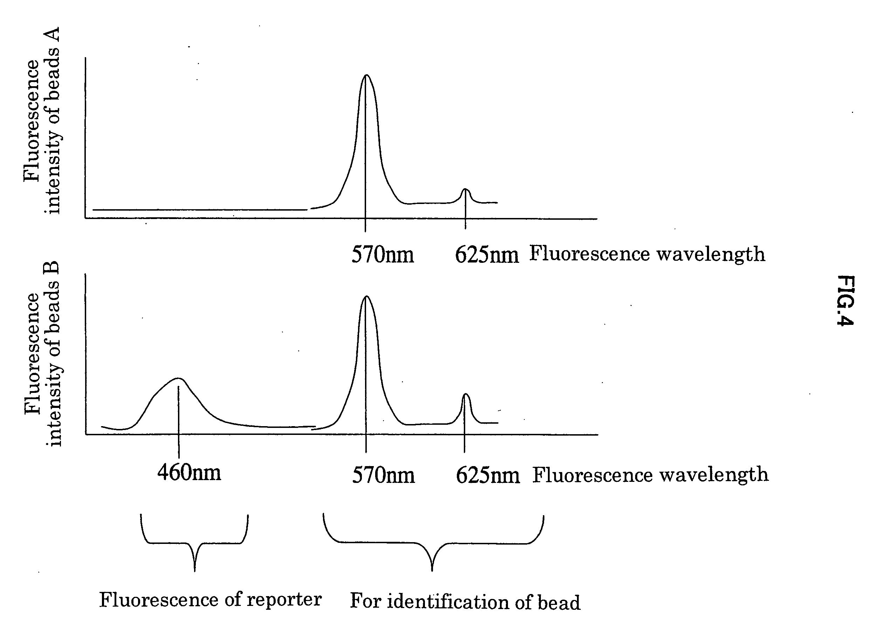

FIG. 4 is a diagram showing the results of measurement by a UV laser-installed FACS (cell sourcer). As seen in the figure, in case of beads B, the fluorescence wavelength at 460nm by a fluorescent substance labeled on a bonded reporter strongly appears. This phenomenon shows that mRNA of β actin gene is existent in total RNA used for the experiment. On the contrary, a phenomenon that fluorescence wavelength of 460nm does not appear at all in case of beads A implies that mRNA of luciferase gene is not expressed in a human body-derived total RNA.

In addition, regarding a gene that expresses only a small amount of mRNA, the quantity of the gene may be generally measured per RT-PCR method. RT-PCR method is a method in which Polymerase Chain Reaction is performed based on cDNA prepared by reverse transcription of mRNA. It is a method to confirm whether or not the gene is existent by amplifying mRNA that expresses in only a small amount in the form of DNA by performing PCR reaction.

It is also possible to perform this RT-PCR method by using DNA bonded to beads as primers. DNA is labeled by using in PCR reaction a biotin-modified dUTP as in a reverse transcription reaction. Thereafter, it is possible to perform a detection by a UV laser-installed FACS by performing secondary labeling using a streptavidin-semiconductor nanoparticles (with particle size of 2.3nm).

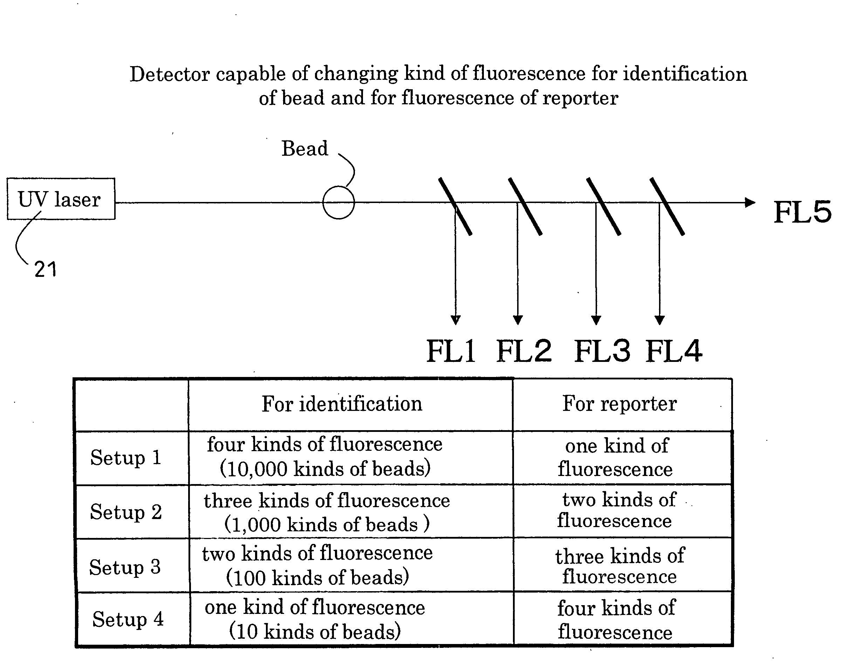

FIG. 5 is a schematic drawing showing a detector capable of changing kind of fluorescence for identification of beads and for detection of reporter. Three kinds of semiconductor nanoparticles have been used up to this stage. It is possible to increase the number of fluorescence to be used for identification of beads or for detection of reporter by further increasing the kinds of the semiconductor nanoparticles. In addition, since ordinary fluorescent reagents have different exciting wavelengths to excite a fluorescent substance if they have different fluorescence wavelengths, it was necessary to predetermine fluorescent substances for identification of beads and for detection of reporter. However, since semiconductor nanoparticles can emit all fluorescence by exciting by a UV laser 21, it is possible to freely switch fluorescence for identification of beads and that for detection of reporter.

For example, if it is assumed that it is possible to identify concentrations in ten steps per one color of fluorescence for identification of beads, it is possible to identify 100 kinds of beads if two colors are used to identify beads. Furthermore, if it is possible to divide fluorescence emitted from each semiconductor nanoparticle by exciting by a UV laser into five levels of wavelength, four kinds of assay methods using beads are assumed to exist as shown in FIG. 5. Namely, in Setting 1, the assay method identifies 10,000 kinds of beads by using four kinds of fluorescence for identification of beads, and detects one kind of reporter by using one kind of fluorescence for detection of reporter. In Setting 2, the assay method identifies 1,000 kinds of beads by using three kinds of fluorescence for identification of beads, and detects two kinds of reporters by using two kinds of fluorescence for detection of reporter. In Setting 3, the assay method identifies 100 kinds of beads by using two kinds of fluorescence for identification of beads, and detects three kinds of reporters by using three kinds of fluorescence for detection of reporter. In Setting 4, the assay method identifies 10 kinds of beads by using one kind of fluorescence for identification of beads, and detects four kinds of reporters by using four kinds of fluorescence for detection of reporter. Thus, it is possible to change to each setting by only performing a software-like control for one hardware configuration.

As for application examples, it can be only judged by ON/OFF to specify SNP. If many kinds of beads are required, Setting 1 is used. Although the quantity of a region to be judged is a small, if assay is to be simultaneously performed on four specimens, Setting 4 should be used.

In addition, the present invention is not limited to the above-mentioned embodiments.

Beads of which surfaces DNA is bonded to may be also semiconductor nanoparticles.

A flow cytometer according to the present invention is materialized in a program to operate a computer as this flow cytometer. This program may be saved in a computer readable record media.

The record media that records this program may be ROM itself inserted into a flow cytometer. Alternatively, it may be a CD-ROM or the like which is readable by inserting the record media into a program reader such as C D-ROM drive provided as an external storage.

In addition, the above mentioned record media may be a magnetic tape, a cassette tape, a floppy disk, a hard disc, MO/MD/DVD, a semiconductor memory or the like.

## Claims

1. Beads, comprising:
semiconductor nanoparticles with a particle size of 1 to 10nm on surfaces thereof.

2. The beads according to claim 1, wherein said semiconductor nanoparticles are composed of semiconductor nanoparticles with a plurality of particle sizes so as to emit a light having peaks at a plurality of wavelengths due to excitation by electromagnetic wave.

3. A method of preparing beads, comprising the steps of:
covalent-bonding DNA with surfaces of beads; and
introducing a label as a reporter onto surfaces of the beads by performing reverse transcription reaction using a desired mRNA as a template, said DNA as a primer, and a base as a material, in which at least one kind of base is labeled.

4. A method of preparing beads, comprising the steps of:
covalent-bonding DNA with surfaces of beads; and
introducing a label as a reporter onto surfaces of the beads by performing Polymerase Chain Reaction using one of a desired mRNA and cDNA as a template, said DNA as a primer, and a base as a material, in which at least one kind of base is labeled.

5. The method of preparing beads according to claim 3, wherein in said step of covalent-bonding, a base with a substituent present at the end thereof is fixed on surfaces of the beads, the beads are filled in a biological reactor, and then the base is further coupled to said substituent, whereby DNA is synthesized on solid phase beads.

6. The method of preparing beads according to claim 4, wherein in said step of covalent-bonding, a base with a substituent present at the end thereof is fixed on surfaces of the beads, the beads are filled in a biological reactor, and then the base is further coupled to said substituent, whereby DNA is synthesized on solid phase beads.

7. A flow cytometer, comprising:
a UV laser emitting a UV laser beam to excite a sample;
a plurality of fluorescence detecting means for detecting a plurality of fluorescence emitted from the sample, respectively; and
a switching means for switching output signals from the fluorescence detecting means to any of beads identifying signal and a reporter detecting signal.

8. A program for allowing a computer to function as a flow cytometer, the flow cytometer comprising:
a UV laser emitting a UV laser beam to excite a sample;
a plurality of fluorescence detecting means for detecting a plurality of fluorescence emitted from the sample, respectively; and
switching means for switching output signals from the fluorescence detecting means to any of beads identifying signal and a reporter detecting signal.
